# EUROPEAN PATENT APPLICATION

(11) **EP 0 689 878 A1**
(43) Date of publication of application: **03.01.1996**
(21) Application number: 95304279.3
(22) Date of filing: 20.06.1995
(51) Int. Cl.: B05B 11/00, B05B 15/00

(54) **Dispensing apparatus**

(30) Priority: 30.06.1994 GB 9413172
(71) Applicant: BESPAK PLC, King's Lynn Norfolk PE30 2JJ (GB)
(72) Inventor: Livingstone, William, Peterborough, Cambridgeshire PE2 6XL (GB)
(74) Representative: Bucks, Teresa Anne

(57) **Abstract**

The invention relates to a dispensing apparatus for delivering an aerosol liquid spray and in particular, but not exclusively, to a device including a displacement pump for spraying pharmaceutical and cosmetic liquid products. The dispensing apparatus comprises an actuating stem (10) defining an outlet duct (11), an inlet portion defining an inlet duct communicating with a reservoir of liquid, an actuator connected to the actuating stem and comprising an atomising spray nozzle defining a nozzle opening for the discharge of liquid, the actuator defining a discharge channel (18) communicating between the outlet duct (11) and the nozzle opening, whereby the inlet duct, the outlet duct (11) and the discharge channel (18) together constitute a delivery channel through which liquid is dispensed in use and in which the apparatus further comprises a filter (21) traversing the delivery channel for the removal of particulate matter from the liquid, said filter (21) comprising a connecting portion (24,25), constituting a connector between the actuating stem (10) and the actuator, and a filtering portion (23).

## Description

This invention relates to dispensing apparatus for delivering an aerosol liquid spray and in particular but not exclusively to a device including a displacement pump for spraying pharmaceutical and cosmetic liquid products.

It is known for liquid aerosol sprays to be produced from hand held dispensers either by action of a displacement pump or a pressurised dispensing container, a tubular actuating stem being depressed to release a pressurised flow of liquid. The liquid is atomised to form an aerosol spray when emerging from a nozzle opening of a spray nozzle provided in an actuator connected to the stem.

It is particularly important in some situations to avoid the accidental discharge in the spray of any particulate material which may be contained in the liquid supply from which liquid is dispensed or which may be present as a contamination of the dispensing apparatus itself. The dispensing of an aerosol spray for inhalation therapy or opthalmic treatment are particular examples where such particulate matter cannot be tolerated.

One way in which particulate matter may contaminate the liquid being dispensed is where the liquid is initially supplied in a glass ampoule which is then fractured in order to gain access to the liquid so that potentially it is possible for fragments of glass to enter the liquid supply.

According to the present invention there is disclosed dispensing apparatus comprising an actuating stem defining an outlet duct, an inlet portion defining an inlet duct communicating with a reservoir of liquid, an actuator connected to the actuating stem and comprising an atomising spray nozzle defining a nozzle opening for the discharge of liquid, the actuator defining a discharge channel communicating between the outlet duct and the nozzle opening, whereby the inlet duct, the outlet duct and the discharge channel together constitute a delivery channel through which liquid is dispensed in use and in which the apparatus further comprises a filter traversing the delivery channel for the removal of particulate matter from the liquid, said filter comprising a connecting portion, constituting a connector between the actuating stem and the actuator, and a filtering portion.

The filter may advantageously be formed as a moulding of porous plastics material.

The moulding may constitute a connector operable to connect separately formed elements of the apparatus so as to function as both filter and connector.

The filter may alternatively comprise a disc shaped membrane of a suitable filter medium such as an acrylic co-polymer membrane.

The membrane may conveniently be supported by a perforate support member to retain the membrane in a configuration in which it traverses the delivery channel.

Preferred embodiments of the present invention will now be described by way of example only and with reference to the accompanying drawings of which:-
Fig. 1 is a part sectioned elevation of a conventional dispensing apparatus;
Fig. 2 is a sectional elevation of a filter in accordance with the present invention for use with apparatus of the type shown in Fig. 1 and comprising a porous plastic moulding; and
Fig. 3 is a sectional elevation of an alternative form of porous plastic moulding.

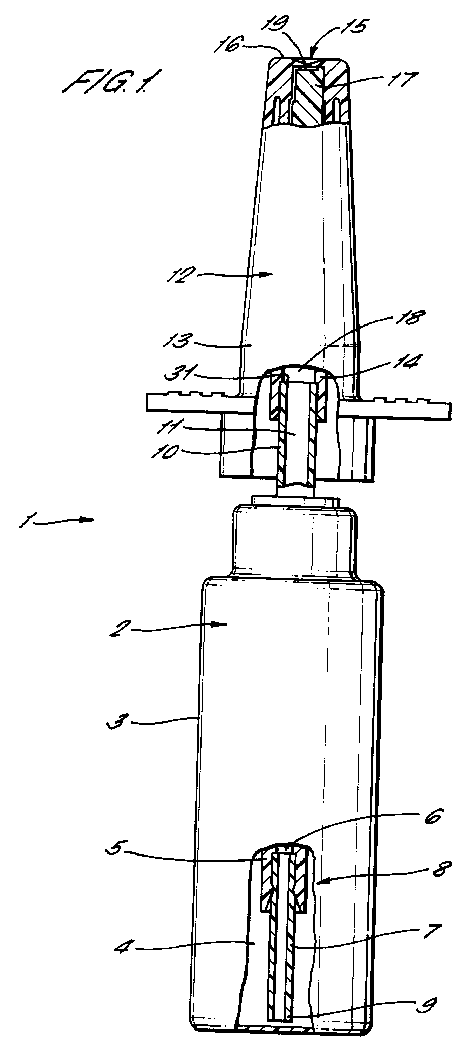

In Fig. 1 a conventional dispensing apparatus 1 comprises a displacement pump 2 connected to a container 3 defining a reservoir 4 within which liquid is received in use.

The pump 2 has a body 5 to which a dip tube 7 is coupled by a connector formation 8, the inlet portion and the dip tube together constituting an inlet portion defining an inlet duct 6. The dip tube 7 has a lower end 9 which is normally submerged within liquid to enable liquid to be drawn by the pump into the inlet duct.

The pump 2 is provided with an actuating stem 10 which projects outwardly from the container 3 and defines an outlet duct 11 which conducts pressurised liquid from the pump 2 in response to actuating movement of the stem in a direction towards the container 3.

An actuator 12 is connected to the stem 10 and comprises an outer member 13 formed as a one piece moulding with a re-entrant cylindrical portion 14 within which the stem is received as a push fit. The cylindrical portion 14 defines a discharge channel 18 which conducts liquid dispensed from the pump via the outlet duct to a nozzle opening 15 provided in an end wall 16 of the outer member 13.

A generally tubular insert 17 is received within the outer member 13 so as to reduce the amount of dead space within the discharge channel 18 and having an end face 19 which co-operates with the end wall 16 to provide swirl inducing formations which serve to induce swirling motion in the liquid immediately prior to ejection from the nozzle opening 15.

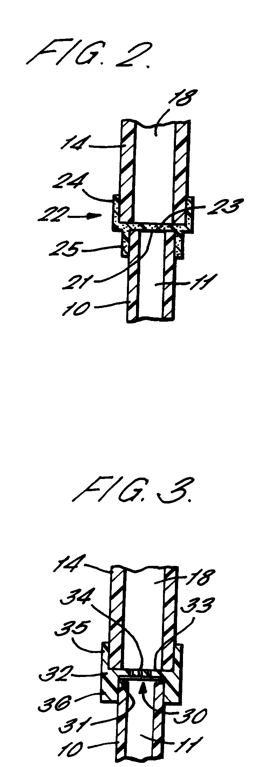

Figs. 2 and 3 illustrate examples of embodiments of the present invention in which corresponding portions of the apparatus of Fig. 1 are replaced by the features of Figs. 2 and 3 in order to constitute in each case a modified apparatus in accordance with the present invention.

In the embodiment of Fig. 2 a filter 21 is constituted by a moulding 22 of porous plastics material, the moulding being formed so as to constitute a connector between the stem 10 and the cylindrical portion 14 of the actuator 12. The moulding 22 comprises a disc portion 23 from which unitarily formed first and second tubular portions 24 and 25 project on opposite sides thereof, thereby defining respective sockets in which the cylindrical portion 14 and the stem 10 are sealingly received as a push fit.

Pressurised liquid dispensed through the outlet duct 11 is thereby constrained to pass through the disc portion 23 before entering the discharge channel 18 and in doing so the filter 21 is thereby operative to remove particulate matter.

In the embodiment of Fig. 3 a filter 30 is constituted by a membrane in the form of a disc which is captively retained between an end face 31 of the stem 10 and a support member 32 constituting a connector between the stem 10 and the cylindrical portion 14 of the actuator 12. The support member 32 comprises a perforate disc portion 33 defining a plurality of aperatures 34 communicating between the outlet duct 11 and the discharge channel 18. The membrane filter 30 is located immediately upstream of the disc portion 33 so as to be supported against movement in the direction of liquid flow in use. The support member 32 further comprises first and second tubular portions 35 and 36 projecting from opposite faces of the disc portion 33 and defining sockets which receive the cylindrical portion 14 of the actuator 12 and the stem 10 respectively.

In use, liquid flowing from the outlet duct 11 into the discharge channel 18 is constrained to pass through the membrane filter 30 and apertures 34 to thereby filter any particulate matter contained in the liquid.

Both embodiments of the invention may alternatively be employed in a dispensing apparatus similar to that illustrated in Fig. 1 but in which displacement pump 2 is replaced by a valve operable to release pressurised liquid from container 3 when the stem 10 is depressed. Such pressurised dispensing containers typically employ a volatile propellant to pressurise the liquid within the container or may alternatively use a compressed gas.

The nozzle shown in Fig. 1 is particularly suitable for nasal administration. Alternative nozzles may be utilised for other purposes such as oral inhalation.

In the embodiment of Fig. 2 the membrane may be bonded to the support member by ultrasonic welding or by the use of a suitable adhesive.

The perforate support member may comprise a disc having a number of circular apertures or may be in the form of a lattice.

The membrane material is preferably hydrophilic.

In a preferred embodiment the membrane is an acrylic co-polymer on a non-woven support with a thickness in the range 0.075 to 0.125 milimetres. The tensile strength of such membranes is typically 3,000 PSI.

The porous plastic moulding may be produced from commonly available polymers such as nylon 6, polycarbonate, PTFE and polypropylene.

The use of such membranes and mouldings is useful in excluding particles having a size greater than 10 microns.

## Claims

1. Dispensing apparatus (1) comprising an actuating stem (10) defining an outlet duct (11), an inlet portion defining an inlet duct (6) communicating with a reservoir (4) of liquid, an actuator (12) connected to the actuating stem and comprising an atomising spray nozzle defining a nozzle opening (15) for the discharge of liquid, the actuator defining a discharge channel (18) communicating between the outlet duct and the nozzle opening, whereby the inlet duct, the outlet duct and the discharge channel together constitute a delivery channel through which liquid is dispensed in use and in which the apparatus further comprises a filter (21,30) traversing the delivery channel for the removal of particulate matter from the liquid, characterised in that said filter comprises a connecting portion (22,32), constituting a connector between the actuating stem and the actuator, and a filtering portion (23).

2. Dispensing apparatus (1) as claimed in claim 1 wherein the filter (21) is a moulding of porous plastics material.

3. Dispensing apparatus (1) as claimed in claim 2 wherein the connecting portion comprises first and second tubular portions (24,25,35,36) projecting from opposite sides thereof and defining respective first and second sockets which respectively receive a cylindrical portion (14) of the actuator and the stem (10).

4. Dispensing apparatus (1) as claimed in any one of the preceding claims wherein the filtering portion (23) comprises a porous disc formed unitarily with the connecting portion (22).

5. Dispensing apparatus (1) as claimed in any one of the preceding claims wherein the filtering portion (23) comprises a disc shaped membrane supported by the connecting portion (22).

6. Dispensing apparatus (1) as claimed in claim 5 wherein the membrane (23) comprises an acrylic co-polymer.

7. Dispensing apparatus (1) as claimed in any of claims 5 or 6 wherein the connecting portion (32) further comprises a perforate support member operable to retain the membrane (23) in a configuration in which it traverses the delivery channel.

8. Dispensing apparatus (1) as claimed in claim 7 wherein the support member comprises a perforate disc (33) portion formed unitarily with the connecting portion (32), the perforate disc portion defining aperatures (34) thereby communicating in use between the outlet duct and the discharge channel.

9. Dispensing apparatus (1) as claimed in any preceding claim comprising a displacement pump (2) actuated by movement of the stem (10) relative to the inlet portion.

10. Dispensing apparatus (1) as claimed in any of claims 1 to 8 comprising a pressurised dispensing container (3) defining the reservoir (14) and further comprising a valve operable to discharge pressurised liquid via the outlet duct (11) in response to depression of the stem (10) relative to the inlet portion.
